# EUROPÄISCHE PATENTANMELDUNG

(11) **EP 1 837 045 A1**
(43) Veröffentlichungstag der Anmeldung: **26.09.2007**
(21) Anmeldenummer: 07005558.7
(22) Anmeldetag: 19.03.2007
(51) Int. Cl.: A61M 1/00

(54) **Absauggerät**

(30) Priorität: 24.03.2006 DE 102006014122
(71) Anmelder: ATMOS MEDIZINTECHNIK GmbH & Co., 79853 Lenzkirch (DE)
(72) Erfinder: Kluge, Stefan, 79254 Oberried (DE); Möller, Mario, 79843 Löffingen (DE)
(74) Vertreter: Westphal, Klaus

(57) **Zusammenfassung**

Es wird ein medizinisches Absauggerät beschrieben, mit welchem Sekrete oder sonstige Flüssigkeiten mit Hilfe einer Vakuumpumpe abgesaugt und in einem Sammelbehälter 10 gesammelt werden. Ein am Deckel 11 des Behälters 10 zentral angeordneter hydrophober luftdurchlässiger Bakterienfilter schützt zum einen die Vakuumpumpe gegen Kontamination und bewirkt zum anderen eine Sperrung des Luftdurchsatzes, wenn Flüssigkeit in die Filteraufnahme 17 gelangt. Ein auf die Filteraufnahme 17 aufgeschobener Sicherungstopf 20 verhindert mit seinen ineinander geschachtelten, die Saugluft umlenkenden Ringkammern 24 und 25, dass bei Schräglage des Behälters 10 bzw. bei Stößen auf diese Sauggut, insbesondere Flüssigkeit, in die Filteraufnahme 17 eintritt.

## Beschreibung

Die Erfindung betrifft ein Absauggerät der im Oberbegriff des Patentanspruchs 1 genannten Art.

Derartige Absauggeräte werden für medizinische Zwecke verwendet, um z.B. bei einem Patienten Sekrete, Flüssigkeiten oder ggf. Erbrochenes abzusaugen. Zu diesem Zweck wird das Absauggut mittels einer Vakuumpumpe in einen Sammelbehälter gesaugt, der nach Befüllung bis zu einer bestimmten Füllhöhe geleert bzw. ausgetauscht werden muss.

Für den Betrieb muss sichergestellt sein, dass Absauggut nicht in die Vakuumpumpe eindringt.

Da beim ambulanten Einsatz, insbesondere im Notfallbereich, häufig nicht sichergestellt ist, dass das Absauggerät in der erforderlichen aufrechten Position in Betrieb ist, besteht die Gefahr, dass Sauggut in die Vakuumpumpe gelangt.

Um dies zu verhindern, ist es erforderlich, bei für den Notfalleinsatz bestimmten Absauggeräten zwischen Vakuumpumpe und dem Sammelbehälter Sicherungsvorrichtungen vorzusehen.

Diesem Zweck dienen schwimmerbetätigte Absperrventile, z.B. Kugelventile, sowie hydrophobe, luftdurchlässige Bakterienfilter.

Im erst genannten Fall ist im Sammelbehälter ein Auftriebskörper vorgesehen, welcher bei kritischem Behälterfüllstand angehoben und das im Luftweg zur Vakuumpumpe angeordnete Absperrventil betätigt.

Eine derartige Sperrvorrichtung ist aber nachteiligerweise au-ßerordentlich lageempfindlich. So sollen derartige Absauggeräte möglichst in aufrechter Position eingesetzt werden. Bei Neigung des Sauggerätes über 90° kommt es schwerkraftbedingt zum Verschluss der im Behälterinnern vorgesehenen Saugöffnung durch den Schwimmkörper, wodurch der Saugbetrieb unterbrochen wird. Nach Aufrichten des Absauggerätes muss das Gesamtsystem belüftet werden, um die Saugöffnung wieder frei zu geben.

Im zweit genannten Fall ist im Absaugweg ein hydrophober, luftdurchlässiger Bakterienfilter vorgesehen, welcher bei Benetzen mit Ansauggut die Verbindung zur Pumpe unterbricht. Da bei medizinischen Absauggeräten ohnehin Bakterienfilter zur Vermeidung einer Kontamination der Vakuumpumpe vorgeschrieben sind, ist an sich diese Lösung günstiger.

Da jedoch derartige Bakterienfilter nicht regenerierbar sind, müssen sie nach Benetzen mit Flüssigkeit ausgetauscht werden, um das Absauggerät weiter benutzen zu können.

Schließlich sind Absauggeräte bekannt, bei welchen der Füllstand des Sammelbehälters mit elektronischen Mitteln kontrolliert und bei Erreichen der maximalen Füllung die Vakuumpumpe abgeschaltet wird. Diese zwar komfortable Lösung ist jedoch nicht nur kostspielig, sondern für den Notfalleinsatz weniger geeignet.

Alle bekannten Geräte sind folglich lageempfindlich, so dass sie für den Notfalleinsatz häufig in unwegsamem, unebenem Gelände wenig geeignet sind.

Der vorliegenden Erfindung liegt darum die Aufgabe zugrunde, ein für den Notfalleinsatz besser geeignetes Gerät zu schaffen, das die oben erläuterten Nachteile nicht besitzt.

Gelöst wird diese Aufgabe gemäß vorliegender Erfindung mit einem Absauggerät, wie es mit Patentanspruch 1 gekennzeichnet ist.

Bei diesem Absauggerät dient ausschließlich der hydrophobe luftdurchlässige Bakterienfilter als Sperre, die wirksam wird, wenn das Füllgut im Sammelbehälter einen vorgeschriebenen Füllstand erreicht hat. Auf ein lageempfindliches Sperrventil ist verzichtet.

Um die eingangs erwähnten, bei hydrophobem Bakterienfilter auftretenden Nachteile zu vermeiden, ist dieser Filter nach dem erfindungsgemäßen Vorschlag innerhalb des Sammelbehälters so angeordnet, dass im Sammelbehälter befindliche Flüssigkeit unabhängig von der Lage des Behälters nicht zum Filter gelangen kann.

Dies wird nach dem Vorschlag gemäß Anspruch 1 konstruktiv im Wesentlichen dadurch erreicht, dass zum einen eine in das Behälterinnere ragende zylindrische Filteraufnahme mittig vorgesehen ist, in welche der gleichfalls zylindrische Filter mit Abstand eingesetzt ist und dass zum anderen auf die Filteraufnahme ein Sicherungstopf aufgesetzt ist, der die von der Einlassöffnung über Absaugöffnungen angesaugte Luft in die Filteraufnahme über labyrinthartig angeordnete Ringkammern leitet.

Konkrete Maßnahmen zur Ausbildung dieser Ringkammern sind Gegenstand der Ansprüche 2 bis 4.

Mittels dieses Sicherungstopfes wird verhindert, dass Flüssigkeit beim Umkippen, Drehen des Behälters oder auch bei Stößen auf diesen in die Filteraufnahme gelangt. Sollten dennoch geringere Flüssigkeitsmengen die im Sicherungstopf vorgesehenen Kammern passieren, können diese in der mit Anspruch 5 angegebenen Sammelkammer aufgefangen werden.

Zur Erzielung gleichmäßiger Luftströme wird gemäß Anspruch 6 empfohlen, die Absaugöffnungen des Sicherungstopfes in gleichmäßigen Abständen auf einem bezüglich der Absaugöffnung der Filteraufnahme konzentrischen Ring anzuordnen, wobei eine optimale Nutzung des Sammelbehältervolumens erreicht wird, wenn, wie ferner vorgeschlagen ist, diese Absaugöffnungen etwa in der räumlichen Mitte des Saugbehälters gelegen sind. Durch diese Maßnahme ist bei einer Befüllung des Sammelbehälters bis zu 70 % der Filter in jeder Lage des Absauggerätes gegen Benetzung mit Flüssigkeit geschützt.

Um eine zu große Saugwirkung und ein Mitreißen von Sauggut an den Absaugöffnungen und innerhalb des Sicherungstopfes zu vermeiden, sollten, wie mit Anspruch 7 vorgeschlagen, die zu durchströmenden Querschnittsflächen, nämlich die Querschnitte der Ein- und Auslaufsöffnungen sowie der Absaugöffnungen und der Ringkammern, so dimensioniert sein, dass die Strömungsgeschwindigkeit der angesaugten Luft 5 m/s nicht überschreitet.

Auch mit dem Vorschlag gemäß Anspruch 8, der sich auf eine unterhalb der Einlassöffnung des Deckels vorgesehene Drallblende bezieht, wird erreicht, dass möglichst kein Sauggut durch Spritzer direkt zu den Absaugöffnungen der Filteraufnahme gelangt. Mit dieser Drallblende wird das die Einlassöffnung passierende Sauggut unter einem Winkel von 90 bis 120° gegen die Behälterwand gelenkt, so dass es nicht ungehindert senkrecht auf die Oberfläche des im Sammelbehälter befindlichen Sauggutes prallt, sondern spiralförmig an der Behälterwand entlang gleitet und mit verminderter Geschwindigkeit bei spitzerem Eintrittswinkel die Oberfläche erreicht. Hierdurch wird nicht nur die Entstehung von Spritzern, sondern auch von Schaum auf der Oberfläche des Behälterinhaltes verhindert.

Falls dennoch, z.B. bei Überfüllung des Sammelbehälters, der hydrophobe Bakterienfilter mit Flüssigkeit benetzt wird und seine Poren sich folglich verschließen, ist er gegen einen neuen Filter auszutauschen. Dieser Austausch gestaltet sich bei einer Konstruktion gemäß Anspruch 9 vergleichsweise einfach.

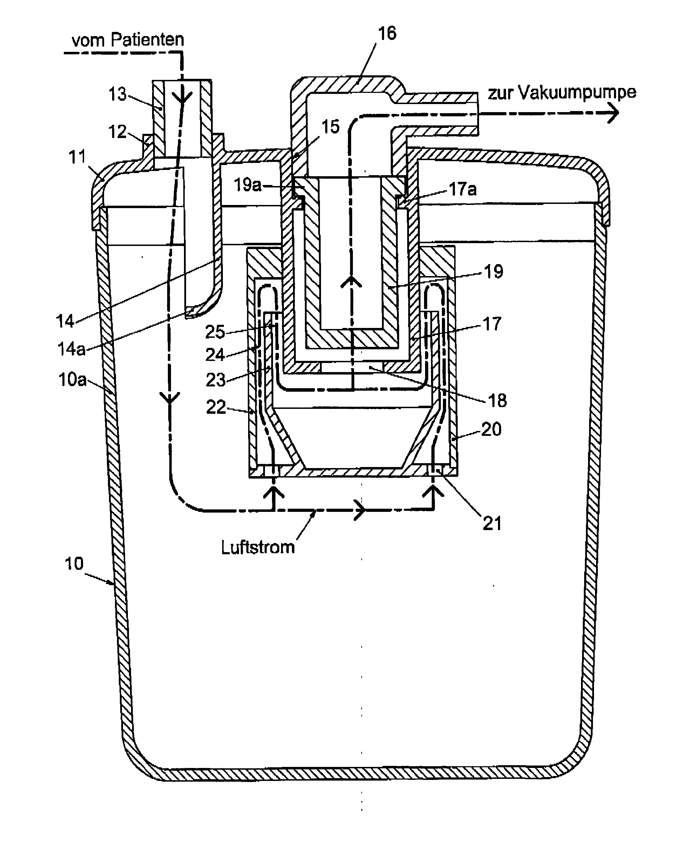

Der. Gegenstand der Erfindung ist nachstehend anhand eines Ausführungsbeispieles im Einzelnen erläutert. Die Zeichnung zeigt im Längsschnitt ein erfindungsgemäß ausgebildetes Absauggerät, wobei der Absaugschlauch, ein sogenannter Patientenschlauch, sowie der Pumpenschlauch und die Vakuumpumpe nicht dargestellt sind.

Das in der Zeichnung veranschaulichte Absauggerät weist einen Sammelbehälter 10 auf, in welchem das Absauggut, also z.B. Sekret, Blut oder sonstige Flüssigkeiten und ggf. Erbrochenes, gesammelt wird. Der Behälter ist mittels eines abnehmbaren Deckels 11 verschlossen. Seitlich weist der Behälter eine Einlassöffnung 12 auf, in welche ein Stutzen für einen nicht dargestellten Absaugschlauch, einen sogenannten Patientenschlauch, eingesetzt ist. Unterhalb der Einlassöffnung 12 in Verlängerung des Stutzens 13 ist eine an die Innenseite des Behälterdeckels 11 angeformte Drallblende 14 vorgesehen. Das untere Ende ist zu einem Krümmer 14a abgebogen. Die Drallblende 14 mit ihrem Krümmer 14a bewirkt, dass den Stutzen 13 passierendes Sauggut gegen die Wand 10a des Sammelbehälters umgelenkt wird.

Zentral am Deckel 11 ist eine in das Behälterinnere ragende zylindrische Filteraufnahme 17 vorgesehen, in welche der hydrophobe, luftdurchlässige Bakterierifilter 19 mit Abstand zur Wandung der Filteraufnahme 17 eingesetzt ist. Der Filter 19 weist an seinem oberen Ende einen Ringflansch 19a auf, mit welchem er auf einem nach innen gerichteten Ringbund 17a der Filteraufnahme 17 aufliegt.

Der Stutzen 16, welcher dem Anschluss des nicht dargestellten und zur Vakuumpumpe führenden Absaugschlauches dient, legt gleichzeitig den Filter 19 innerhalb der Filteraufnahme 17 fest.

An ihrem im Behälterinnern gelegenen Ende weist die Filteraufnahme 17 eine Absaugöffnung 18 zum Eintritt der Saugluft auf.

Entscheidend für die erfindungsgemäße Lösung ist der Sicherungstopf 20, welcher das untere Ende der Filteraufnahme 17 umgibt. Er besitzt zwei konzentrische, ineinander geschachtelte Ringkammern, nämlich eine äußere Ringkammer 24 und eine innere Ringkammer 25, welche koaxial zur Filteraufnahme 19 angeordnet sind. Diese Ringkammern sind zum einen aus dem Außenring 22 und dem Innenring 23 und zum anderen aus dem Innenring 23 und der äußeren Mantelfläche der zylindrischen Filteraufnahme 17 gebildet. Sie sind labyrinthartig so angeordnet und ausgebildet, dass durch die Absaugöffnung 21 eintretende Luft, wie mit der strichpunktierten Linie angedeutet, zunächst in Richtung auf die Auslassöffnung 15 eintritt und dann im oberen Teil umgelenkt wird, um in entgegengesetzter Richtung radial zu der Absaugöffnung 18 der Filteraufnahme 17 geleitet zu werden. Bei einer derartigen Gestaltung des Sicherungstopfes ist wirksam verhindert, dass Flüssigkeit in beliebiger Schräglage, ja sogar bei Überkopfstellung, in die Filteraufnahme 17 gelangt, soweit jedenfalls der Behälter 10 nur bis zu 70 % seines Volumens gefüllt ist.

Etwaige Spritzer, die doch noch von dem Luftstrom mitgerissen werden, werden in der unterhalb der Filteraufnahme 17 vorgesehen Sammelkammer 26 aufgefangen. Diese Sammelkammer 26, die trichterförmig ausgebildet ist, ist von den unteren Enden 23a des Innenringes 23 gebildet. Der Sicherungstopf 20 ist wie die Filteraufnahme 17 und der Filter 19 im Wesentlichen zylindrisch ausgebildet. Die am unteren Ende des Sicherungstopfes 20 vorgesehenen Absaugöffnungen 21 liegen etwa in der räumlichen Mitte des Saugbehälters 10. Sie sind in gleichmäßigen Abständen voneinander auf einem in der Schnittdarstellung nicht erkennbaren Lochkreis positioniert.

### Bezugszeichenliste

- 10: Sammelbehälter
- 10a: Wand
- 11: Deckel
- 12: Einlassöffnung
- 13: Stutzen für Absaugschlauch
- 14: Drallblende
- 14a: Krümmer
- 15: Auslassöffnung
- 16: Stutzen für Pumpenschlauch
- 17: Filteraufnahme
- 17a: Auflagering
- 18: Absaugöffnung
- 19: Bakterienfilter
- 19a: Ringflansch
- 20: Sicherungstopf
- 21: Absaugöffnung
- 22: Außenring
- 23: Innenring
- 23a: unterer Bereich des Innenrings
- 24: äußere Ringkammer
- 25: innere Ringkammer
- 26: Sammelkammer

## Patentansprüche

1. Absauggerät, bestehend aus einem Sammelbehälter mit einem abnehmbaren Deckel, der eine Einlassöffnung für einen Absaugschlauch und eine Absaugöffnung für einen Pumpenschlauch aufweist, wobei im Strömungsweg vor der Absaugöffnung ein austauschbarer, hydrophober, luftdurchlässiger Bakterienfilter angeordnet ist,
**dadurch gekennzeichnet, dass** am Deckel (11) mittig eine in das Behälterinnere ragende, zylindrische Filteraufnahme (17) vorgesehen ist, dass in die Filteraufnahme (17) der gleichfalls zylindrische Filter (19) mit Abstand eingesetzt ist und dass auf die Filteraufnahme (17) ein Sicherungstopf (20) aufgesetzt ist, der die von der Einlassöffnung (12) angesaugte Luft in die Filteraufnahme (17) über Absaugöffnungen (21) und Ringkammern (24, 25) leitet, die derart labyrinthartig angeordnet sind, dass im Sammelbehälter (10) befindliches Sauggut unabhängig von der Lage des Behälters (10) nicht in die Filteraufnahme (17) gelangt.

2. Absauggerät nach Anspruch 1,
**dadurch gekennzeichnet, dass** der Sicherungstopf (20) eine äußere und eine innere Ringkammer (24, 25) aufweist, die in Bezug auf die Filteraufnahme (17) koaxial angeordnet sind, wobei die äußere Ringkammer (24) nach unten gerichtete Absaugöffnungen (21) aufweist und an ihrem gegenüberliegenden Ende nach Umlenkung des Luftstromes um etwa 180° in die innere Ringkammer (25) übergeht, welche mit der Absaugöffnung (15) der Filteraufnahme (17) verbunden ist.

3. Absauggerät nach Anspruch 1 oder 2,
**dadurch gekennzeichnet, dass** im Sicherungstopf (20) eine Sammelkammer (26) unterhalb der Filteraufnahme (17) vorgesehen ist, in welche die innere Ringkammer (25) mündet.

4. Absauggerät nach Anspruch 1, 2 oder 3,
**dadurch gekennzeichnet, dass** der Sicherungstopf (20) einen Außen- und einen Innenring (22, 23) aufweist, welche zusammen mit der Filteraufnahme (17) die äußere und die innere Ringkammer (24, 25) begrenzen.

5. Absauggerät nach einem der Ansprüche 1 bis 4,
**dadurch gekennzeichnet, dass** der Innenring (23) des Sicherungstopfes (20) in seinem unteren Bereich (23a) die Sammelkammer (26) bildet und trichterförmig ausgebildet ist.

6. Absauggerät nach einem der Ansprüche 1 bis 5,
**dadurch gekennzeichnet, dass** die Absaugöffnungen (21) des Sicherungstopfes (20) in gleichmä-ßigen Abständen auf einem bezüglich der Absaugöffnung (18) der Filteraufnahme konzentrischen Kreis angeordnet sind und etwa in der räumlichen Mitte des Saugbehälters (10) gelegen sind.

7. Absauggerät nach einem der Ansprüche 1 bis 6,
**dadurch gekennzeichnet, dass** die Querschnitte der Einlass- und Auslassöffnungen (12, 15) sowie der Absaugöffnungen (18, 21) und der Ringkammern (24, 25) so dimensioniert sind, dass die Strömungsgeschwindigkeit der angesaugten Luft 5 m/s nicht überschreitet.

8. Absauggerät nach einem der Ansprüche 1 bis 7,
**dadurch gekennzeichnet, dass** unterhalb der Einlassöffnung (12) des Deckels (11) eine das Sauggut um etwa 90° bis 120° nach außen gegen die Wand (10a) des Sammelbehälters (10) umlenkende Drallblende (14) vorgesehen ist.

9. Absauggerät nach einem der Ansprüche 1 bis 8,
**dadurch gekennzeichnet, dass** die Filteraufnahme (17) einen nach innen gerichteten Auflagering (17a) aufweist, auf welchem der Filter (19) mit einem Ringflansch (19a) aufliegt, welcher nach oben durch den in die Auslassöffnung (15) des Deckels (11) eingesetzten Stutzen (16) für den Pumpenschlauch gesichert ist.
